# EUROPEAN PATENT APPLICATION

(11) **EP 4 736 801 A1**
(43) Date of publication of application: **06.05.2026**
(21) Application number: 24306848.3
(22) Date of filing: 31.10.2024
(51) Int. Cl.: A61B 34/30, A61B 34/00

(54) **ROBOTIC CATHETER MANIPULATOR**

(71) Applicant: Université Marie et Louis Pasteur, 25000 Besançon (FR); École Nationale Supérieure de Mécanique et des Microtechniques, 25030 Besançon Cedex (FR); Centre National de la Recherche Scientifique, 75016 Paris (FR); Centre Hospitalier Régional Universitaire de Besançon, 25000 Besançon (FR)
(72) Inventor: RABENOROSCOA, Kanty, Couchapon (FR); CHARBONNIER, Guillaume, Besançon (FR); ROUX, Pierre, Besançon (FR); HAOUAS, Wissem, Besançon (FR); ROUGEOT, Patrick, Besançon (FR); BIONDI, Alessandra, Paris (FR); MOULIN, Thierry, Besançon (FR)
(74) Representative: Bandpay & Greuter

(57) **Abstract**

The disclosure notably relates to a robotic catheter manipulator that comprises a pneumatic gripper, the pneumatic gripper providing a first state for gripping a catheter and a second state for releasing the catheter, and a positioning system on which the pneumatic gripper is maintained.

## Description

### TECHNICAL FIELD

The disclosure relates to the field of robotic catheter manipulators for interventional neuroradiology.

### BACKGROUND

Interventional neuroradiology (IN) or neurointervention or endovascular neurosurgery uses medical imaging and minimally invasive technologies to treat vascular conditions involving the central nervous system, especially the brain. Interventional neuroradiology procedures are effective and safe, particularly for treating acute ischemic stroke (AIS) also referred to as strokes or brain attacks. An AIS is an interruption of cerebral blood flow. A stroke occurs when a blockage in an artery prevents blood from reaching cells in the brain. Interventional neuroradiology is also used to treat conditions hemorrhagic strokes caused by aneurysms, arteriovenous malformations (AVMs), and other vascular abnormalities in the brain.

Neurointerventions procedures are generally done in an angio suite or cath-lab, similar to an operating room, but with special X-ray equipment (e.g. live fluoroscopy, digital subtracted angiography, 3D rotational imaging capability), which allows the physician to precisely navigate medical devices insides the human vasculature.

Minimally invasive technologies comprise the use of interventional tools such as guidewires and catheters. Guidewires are primarily a navigation tool used to create a pathway, while catheters are the instrument that perform the actual medical procedure, such as delivering treatment or performing an intervention. Guidewires allow operators to traverse along a given vessel or along a track of vessels. A guidewire is a thin, flexible wire that is used to enter small and tortuous vessels to act as a guide for subsequent insertion of another instrument such as a microcatheter or balloon catheter.

Catheters used for neuro interventions are specifically designed for navigating the complex and delicate blood vessels of the brain. A typical schematic of a full range of neuro-interventional products for a neurointervention comprises one or more guide catheters that are large and robust catheters that allow smaller catheters to pass through and facilitate the placement of various medical devices while providing stability during the procedure, e.g.. It can further comprise one or more intermediate catheters, each being designed to provide stability by reducing the tendency of the microcatheter to move during delivery/implantation of medical devices. It also comprises a microcatheter with a small diameter that can be tracked over a guidewire in order to reach the target lesion; the microcatheter may be replaced by a balloon catheter, which is a soft catheter with an inflatable balloon at its tip. The set of robust, intermediate and micro catheters form a system of telescopic catheters.

A neurointervention involves inserting the system of telescopic catheters into a peripheral artery, e.g. the inguinal crease. The guide catheter is navigated to the origin of the internal cervical carotid artery. An intermediate catheter is inserted in the guide catheter and navigates the proximal intracranial vessels. Finally, a microcatheter is inserted which navigates more distally in the intracranial vessels. The navigation catheters may use guidewires that can be telescopically mounted too.

The handling of these telescopically mounted catheters/guides is not made possible for robotic assistants that have been developed for interventional cardiology. These robotic assistants allow catheters to be manipulated using cables or magnetic actuation, but they all suffer a major drawback that is they can only manipulate a microcatheter and a micro guide. In other words, they are not able to manipulate telescopically mounted catheters or guides. In addition, these robotic assistants can only handle a limited number of catheters and guides whereas a wide range of catheters and guides in terms of diameter, length, flexibility... exist.

For these reasons, none of these robotic assistants can perform a complete interventional neuroradiology procedure as they cannot handle more than two telescopically mounted catheters.

Within this context, there is still a need for an improved robotic catheter manipulator in interventional neuroradiology procedure.

It is therefore provided a robotic catheter manipulator that comprises a pneumatic gripper, the pneumatic gripper providing a first state for gripping a catheter and a second state for releasing the catheter; and a positioning system on which the pneumatic gripper is maintained.

The method may comprise one or more of the following:
- the pneumatic gripper comprises a cavity adapted to receive the catheter on a portion thereof, the cavity forming a wall around the portion of the catheter, the wall comprising one or more pneumatic cushions; and
   wherein the pneumatic gripper provides the first state when the one or more pneumatic cushions are inflated and provides the second state when the one or more pneumatic cushions are deflated;
- the one or more pneumatic cushions are fluidly interconnected;
- the pneumatic gripper has an elongated shape, preferably the elongated shape is a cylinder; and wherein the cavity adapted to receive the catheter on a portion thereof has an elongated shape with a diameter comprises between 1mm and 3mm, preferably between 1.5mm and 1.85mm;
- the cavity is substantially a cylindric, the cavity form a through-hole, and the wall comprising one or more pneumatic cushions has a thickness which is substantially the same along its length;
- an inlet and an outlet fluidly connected with the pneumatic gripper, preferably the inlet and outlet being merged;
- the positioning system comprises a first actuator for displacing the pneumatic gripper about a first axis and a second actuator for rotating the pneumatic gripper about the first axis;
- the robotic catheter manipulator further comprises a body comprising at least one guide forming the first axis, the pneumatic gripper being movable along the guide;
- the first and second actuators comprise compressed-air stepper motors or others electric motors;
- the robotic catheter manipulator further comprises a clamp for securing one end of the catheter.

It is also provided a method of robotically guiding a catheter. The method comprises:
- providing the above robotic catheter manipulator;
- introducing a catheter in the pneumatic gripper;
- commanding, by an operator, the positioning system to:
   -- provide the first state for gripping the catheter;
   -- command positioning system to translate and/or rotate the pneumatic gripper from a first position to a second position;
   -- command the positioning system to provide the second state for releasing the catheter; and
- repeating the commanding of the positioning system until the catheter is positioned as requested by the operator.

The method may comprise one or more of the following:
- the catheter is a telescopic catheter comprising two or more tubes with a range of diameters that fit within each other, further comprising providing a robotic catheter manipulator for each of the two or more tubes; commanding successively, by an operator, the positioning system for each tube of the telescopic catheter, starting with the largest diameter tube and ending with the smallest diameter tube.

It is further provided a robotic catheter placement system comprising:
- one or more robotic arms providing up to six degrees of freedom, each of the one or more robotic arms comprising one above robotic catheter manipulator;
- a command unit for configuring:
   -- the position of the one or more robotic arms;
   -- the selection of the first and second states for each of the said one robotic catheter manipulator; and
   -- the positioning system.

The system may comprise one or more of the following:
- the control unit comprises a processing unit and a memory storing instructions that, when executed by the processing unit, cause the command unit to configure the position of the one or more robotic arms, configure the selection of the first and second states for each of the said one robotic catheter manipulator, and configure the positioning system.

It is further provided a computer program comprising instructions for causing in real-time the above command unit to configure the position of the one or more robotic arms, configure the selection of the first and second states for each of the said one robotic catheter manipulator, and configure the positioning system.

It is further provided a computer readable storage medium having recorded thereon the computer program.

### BRIEF DESCRIPTION OF THE DRAWINGS

Non-limiting examples will now be described in reference to the accompanying drawings, where:
- FIG. 1 shows a schematic representation of an example of the robotic catheter manipulator;
- FIG. 2 shows a schematic representation of the positioning system of an example of the robotic catheter manipulator;
- FIG. 3 shows a screenshot of a computer-aided design an example of the robotic catheter manipulator;
- FIGs. 4a-b, FIGs. 5a-b, FIGs. 6-9 show examples of pneumatic grippers;
- FIG. 10 shows an example of a method of robotically guiding a catheter;
- FIGs. 11-14 show an example of robotic catheter placement system; and
- FIG. 15 shows an example of control unit.

### DETAILED DESCRIPTION

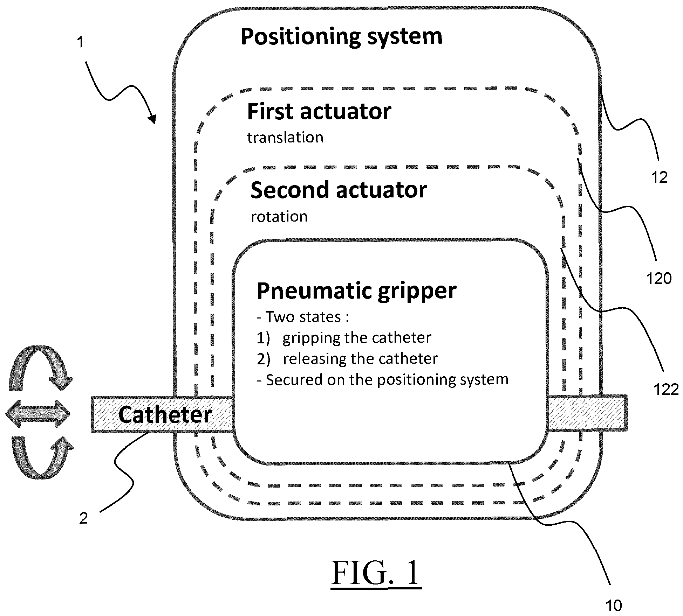

With reference to the schematic representation of **FIG. 1****,** it is proposed a robotic catheter manipulator 1. The manipulator is for driving one or more elongated medical devices. Elongated medical device refers to, but is not limited to, a rod shape medical device, catheters, guide catheters , microcatheters, balloon catheters, stent catheters, wire-based devices such as guidewires, embolization coils, stent retrievers..., and medical devices that are any combination of these. It the following of the description, these terms are considered as synonyms.

Robotic manipulator for elongated medical device (e.g. catheter) means a device that is robotically and operatively controlled to rotate the elongated medical device about its longitudinal axis, to pinch and/or unpinch the elongated medical device, and/or to translate the elongated medical device along its longitudinal axis. The longitudinal axis of the elongated medical device may be defined by a proximal portion of the elongated medical device to a distal portion of the elongated medical device. For example, a catheter comprises distal portion that is inserted in the blood vessels of the patient and proximal portion that is kept outside the blood vessels; the catheter can be seen as a straight device from which the longitudinal axis can be inferred.

In examples, the elongated medical devices are telescopically mounted, e.g. telescopically mounted catheters and/or guides. Telescopically mounted the elongated medical device consists of multiple sections or sheaths that can slide within each other, much like the segments of a telescope. This design allows the elongated medical device to be inserted into the body in a controlled and precise manner, often in stages. Telescopically mounted elongated medical devices (e.g. telescopically mounted catheters) are used in neurointerventions. As known in the art, telescopically mounted catheters comprises one or more guide catheters that are large and robust catheters, one or more intermediate catheters that may be designed to provide stability of the microcatheter, and microcatheter (or any other tool such a balloon catheter, a stent catheter...) with a small diameter to reach the target lesion.

The robotic catheter manipulator comprises a pneumatic gripper 10. The pneumatic gripper provides a first state for gripping a catheter and a second state for releasing the catheter.

The pneumatic gripper uses a compressed fluid to operate and control a gripping action. The fluid may be, but is not limited to, air or other gases such as inert gases like nitrogen. The fluid may be a hydraulic fluid such as, but not limited to, mineral oil-based fluid, synthetic fluid, water-based fluid...

The pneumatic gripper provides two states, the first state is for gripping a catheter and the second state is for releasing a catheter. Gripping means that the pneumatic gripper pinches the catheter; the pneumatic gripper comes into contact with the catheter to be handled. Releasing means that the pneumatic gripper unpinch the catheter; the pneumatic gripper is no more in contact with the catheter that was handled.

The first and second states are triggered by the presence or the absence of the compressed fluid. In examples, the first state is triggered when the fluid that is admitted in pneumatic gripper and compressed; on the contrary, the second state is triggered when the fluid that is admitted is decompressed. Hence, the first and second states provided by the pneumatic gripper are typically controlled by regulating the flow of compressed air in the pneumatic gripper, e.g. via valves. By controlling the air pressure and flow, the force and speed of the gripping action can be adjusted.

The robotic catheter manipulator comprises a positioning system 12 on which the pneumatic gripper is maintained. The positioning system aims at displacing the pneumatic gripper along a longitudinal axis of the elongated medical device (e.g. a catheter) and/or around the longitudinal axis of the elongated medical device. The positioning system can thus perform changes of positions of the elongated medical device (e.g. a catheter) about a referential, where the referential may be a referential attached to the body of the patient subject of the neurointervention. The positioning system 12 will be rediscussed latter.

The robotic catheter manipulator of the present disclosure improves the manipulation of an elongated medical device such as a catheter. Firstly, the pneumatic gripper improves the gripping and the release of the elongated medical device as the pneumatic gripper can be easily adapted to any type of elongated medical device in terms of diameter, length, flexibility... For example, no special nesting is required to fix and/or grip and/or ungrip (that is, release) a catheter, thus making the pneumatic gripper universal for all types of elongated medical device. This makes it possible to use the same pneumatic gripper to be used on telescopic catheters that can comprise up to (and even more) three catheters with different diameter, length, flexibility parameters. The same applies to telescopic guidewires. Therefore, a robotic catheter placement system can use the same universal robotic catheter manipulators, and each of the robotic catheter manipulators can handle any part of the telescopic elongated medical device, thus making possible cooperation between the robotic catheter manipulators, not also making easier the construction and the maintenance of the robotic catheter placement system.

The robotic catheter manipulator of the present disclosure also improves the manipulation of the elongated medical device as the pneumatic gripper allows reproducing the gestures that are performed by a human that would manipulate the elongated medical device. Indeed, the pneumatic gripper takes the form of a flexible gripper able to operate around the catheter, e.g. as a cuff around the catheter, thus reproducing the fingers of a human manipulator. The pneumatic actuator can mimic the thumb-index pinch, while known solutions of the prior art rather rely on friction-based solutions. One of the advantages is that it allows infinite catheter travel, but minimal actuation of the pneumatic gripper.

Further advantages of the present invention will be discussed herein after.

In examples, the pneumatic gripper may comprise a cavity adapted to receive the catheter on a portion thereof. Here "cavity" refers to a hollow or void space within the pneumatic gripper. In other words, the cavity can accommodate a portion of the catheter. The cavity forms a wall around the accommodated portion of the catheter. Here around the accommodated portion of the catheter means that the accommodated portion of the catheter is at least partially surrounded by the wall. The wall comprises one or more pneumatic cushions, where a pneumatic cushion is a structure that uses air or gas or any other fluid under pressure to inflate or deflate; the pneumatic cushion provides gripping of the elongated medical device when inflated, and provides a release of the gripping of the elongated medical device when deflated, or conversely.

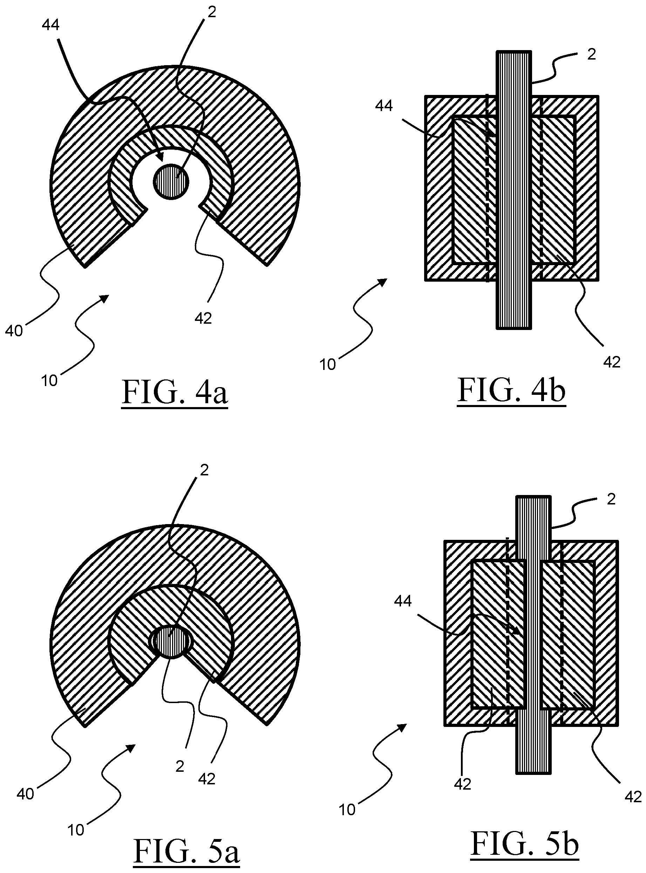

FIG. 4a and 4b illustrate an example of a pneumatic gripper 10. FIG. 4a is a top view and FIG. 4b a front view of the pneumatic gripper 10. The pneumatic gripper 10 comprises a body 40 where the body forms a cavity 44 adapted to receive an elongated medical device 2. By adapted to receive an elongated medical device 2 means that the dimensions of cavity are sufficient for the elongated medical device 2 not to be in contact with the wall of the cavity when the pneumatic gripper provides the second state (the catheter is released or ungripped). As illustrated on FIG. 4a, the cavity 44 forms a wall around a portion of the elongated medical device 2. The wall does not completely surround the elongated medical device 2 as the cavity has an aperture. The wall formed by the cavity comprises in this example one pneumatic cushion 42, being understood that it might comprise two or more pneumatic cushions. When the one or more pneumatic cushions are deflated, pneumatic gripper provides the second state where the catheter is released, or conversely. FIG. 4b shows that only a portion of the elongated medical device 2 is accommodated in the cavity 44 as the elongated medical device 2 is longer that the pneumatic gripper 10.

FIG. 5a and 5b illustrate the example of the pneumatic gripper 10 of FIG.s 4a and 4b, but with the cushion 42 that is inflated so that the pneumatic gripper provides the first state where the elongated medical device 2 is gripped. One can notice on FIG. 5b that only part of the elongated medical element 2 that is pinched (or gripped) by the pneumatic gripper 10 is in contact with the cushion 42: indeed, the cavity is not closed and comprises an aperture.

On FIGs. 4a, 4b, 5a and 5b, the dimensions of the represented elements have been exaggerated for illustration purposes only. For example, the body 40 may be much thinner; the same for the cushion 42. In these examples, the body and the cushion form two separate elements. It is to be understood that the body 40 and the cushion 42 may form one single element, e.g. the walls forming the cavity 44 of the body 40 may be the cushion 42. The examples of FIGs. 4a, 4b, 5a and 5b show only one cushion, being understood that several cushions might be used instead of only one.

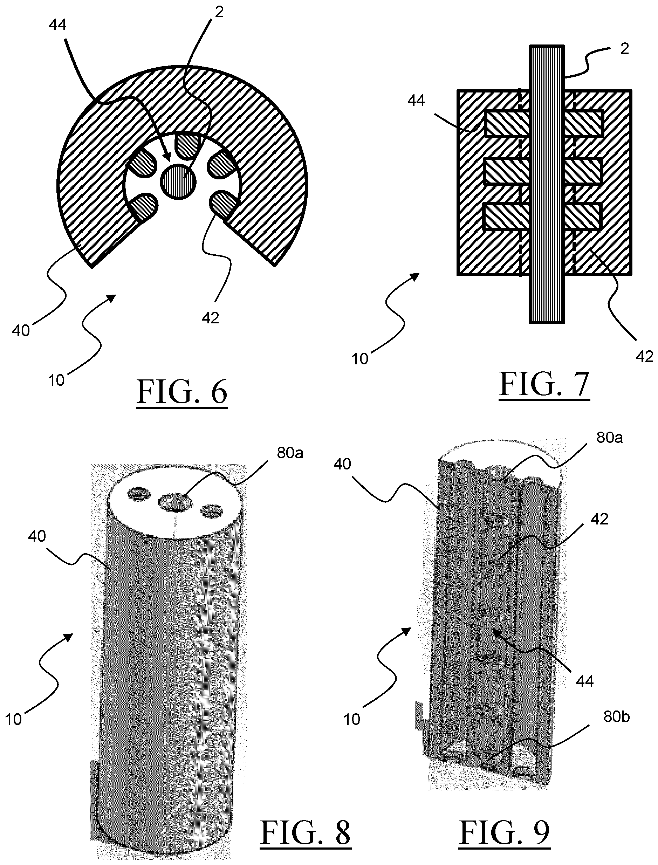

FIGs. 6 and 7 are examples similar to those of FIGs. 4a, 4b, 5a and 5b, except that the pneumatic gripper 10 comprises several cushions comprised in the wall formed by the cavity 44. On FIG. 6, the pneumatic gripper 10 comprises five cushions that are arranged in the longitudinal direction of the pneumatic gripper 10. On FIG. 7, the pneumatic gripper 10 comprises three cushions that are arranged perpendicular to the longitudinal direction of the pneumatic gripper 10. The number of cushions may vary depending on the configuration. The use of several cushions allows a variation of the friction between the cavity and the elongated medical device (e.g. a rod shape medical device), thereby improving the reproduction of the gestures that are performed by a physician during an a procedure such as a neurointervention.

In examples, a special surface treatment can be applied on the one or more cushions to guarantee and/or improve the grip of the elongated medical device.

In examples, the pneumatic cushions may be fluidly interconnected. This means that the inflation and deflation of all the cushions can be performed through one of the cushions, thus making easier the management of the pneumatic gripper and the transition from the first to second states, or inversely.

In examples, the pneumatic gripper may have an elongated shape; the elongation is along the longitudinal axis of the elongated medical device. This is particularly adapted for the manipulation of the elongated medical devices: indeed, the elongated medical device is held by the pneumatic gripper over a greater length, which ensures a grip effect distributed over a greater length and therefore enables superior holding, while at the same time being able to reduce the pressure exerted by the cushions at the contact points between the elongated medical device and the wall of the cavity, e.g. the cushions.

In examples, the length of the elongated shape may be comprised between 0.5cm and 15cm; the cavity accommodating the elongated medical device may have substantially the same length. In examples, the pneumatic gripper may have a shape of a cylinder, but not limited to.

In examples, the cavity may have an elongated shape with a diameter comprised between 1mm and 3mm; the dimension (the diameter) of the cavity allows the accommodation of an elongated medical device with a diameter smaller that 3mm. In an example, the dimension (the diameter) of the cavity is comprised between 1.5mm and 1.85mm; it allows the accommodation of an elongated medical device with a diameter smaller than 1,85mm.

In examples, the cavity may be substantially a cylinder. For example, the cavities represented in the examples of FIGs. 4a, 4b, 5a and 5b have substantially the shape of a cylinder. The accommodation of the elongated medical device in the cavity is made easier as the elongated medical device is substantially a cylinder too.

In examples, the cavity may be a through hole. The through hole may have substantially the shape of a cylinder. A cavity being a through hole, the elongated medical device cannot leave de cavity via an aperture of the cavity; the elongated medical device is therefore controllable by the pneumatic gripper at any time. In these examples, the wall comprising the one or more pneumatic cushions has a thickness which is substantially the same along its length. This ensures that the elongated medical device has not contact with any point of the wall when the pneumatic gripper provides the second state for releasing the elongated medical device.

FIGs 8 and 9 show an example of a pneumatic gripper 10. FIG. 9 is a sectional view of FIG. 8. The body 40 of the pneumatic gripper 10 has an elongated shape, which is a cylinder. The pneumatic gripper comprises a cavity 44 forming a through hole in the body of the pneumatic gripper; the through hole has substantially the shape of a cylinder. Two apertures 80a and 80b give access to the cavity. In these examples, the wall of the cavity comprises several cushions 42 that are fluidly interconnected. In these examples, the inner diameter of the cavity is 1.85 mm; the cavity is designed to accommodate A catheter with an outer diameter of 1.67mm. The clearance attributed to this dimension has been refined by manufacturing trials. Hence, the cylindric cavity provides a larger contact surface to the elongated medical device and the wall thickness of the pneumatic gripper is homogeneous throughout the inner tube as the body and the cavity have substantially a cylindric shape; the cushions comprised in the wall are evenly distributed so that so deformations on the elongated medical device are distributed and no rigidity is localized.

Still in the examples of FIG. 9, the cushions, when inflated, form a crenellated shape in contact with the elongated medical device. The crenellated shape provides good performances in terms of gripping of the elongated medical device while limiting the volume of fluid for inflating the cushions. The crenellated shape increases the friction between the cavity and the elongated medical device (e.g. a rod shape medical device), thereby obtaining an improved grip of the elongated medical device by the robotic catheter manipulator. It is to be understood that the crenellated shape of the cushions can be used with any of the examples discussed hereinabove, e.g. as represented on FIGs. 6 and 7.

Referring back to FIG. 1, examples of the positioning system 12 are discussed. The position system aims at displacing the pneumatic gripper 10 for reproducing the gestures that are performed by a physician during a neurointervention. The following three motions can usually be performed by the robotic catheter manipulator. The push and pull (or retract) is the gesture that corresponds to the forward and backward movement of the elongated medical device in the blood vessels. The rotation corresponds to changes of the orientation of the elongated medical device in blood vessels, then followed by pushing to change the direction. The push and turn coordination corresponds to vessel navigation and lesion location that are performed by simultaneously pushing and rotating the elongated medical device.

Thus, the positioning system can perform changes of positions of the elongated medical device (e.g. a catheter), when gripped by the pneumatic gripper, about a referential, where the referential may be a referential attached to the body of the patient subject of the neurointervention.

In examples, the positioning system may comprise a first actuator 120 for displacing the positioning system about a first axis and a second actuator 122 for rotating the positioning about the first axis. The first axis may be the longitudinal axis defined by the elongated medical device (e.g. a catheter). The first axis may be defined by the pneumatic gripper 10, e.g. the elongated cavity defines the first axis.

The first actuator 120 thus performs a translation of the pneumatic gripper in a direction going from a proximal portion of the elongated medical device to a distal portion of the elongated medical device; the direction forms the longitudinal axis or first axis. For example, the positioning system can translate the pneumatic gripper from a first to a second position where the second position is closer to the body of the patient subject of the neurointervention. By extension, and when the pneumatic gripper is in the first state (the catheter is pinched or gripped), the translation movement from the first to the second positions can thus correspond to a displacement of the distal portion of the elongated medical device (e.g. a catheter) into the blood vessels the patient subject of the neurointervention or on the contrary to the distancing of the distal portion of the elongated medical device (e.g. a catheter) to the body of the patient subject of the neurointervention, due to the application of a force by the positioning system.

The second actuator 122 thus performs a rotation of the pneumatic gripper by using the longitudinal axis (the first axis) as rotation axis. For example, the positioning system can rotate the pneumatic gripper from a third position to a fourth position where the fourth position is a change in angular orientation of the pneumatic gripper about longitudinal axis. The rotational movement from the third to the fourth positions can thus correspond to clockwise or counterclockwise rotation of pneumatic gripper. By extension, and when the pneumatic gripper is in the first state (the catheter is pinched or gripped), the rotational movement from the third to the fourth positions can thus correspond to clockwise or counterclockwise rotation of the elongated medical device (e.g. a catheter) about the longitudinal axis due to an applied torque by the positioning system.

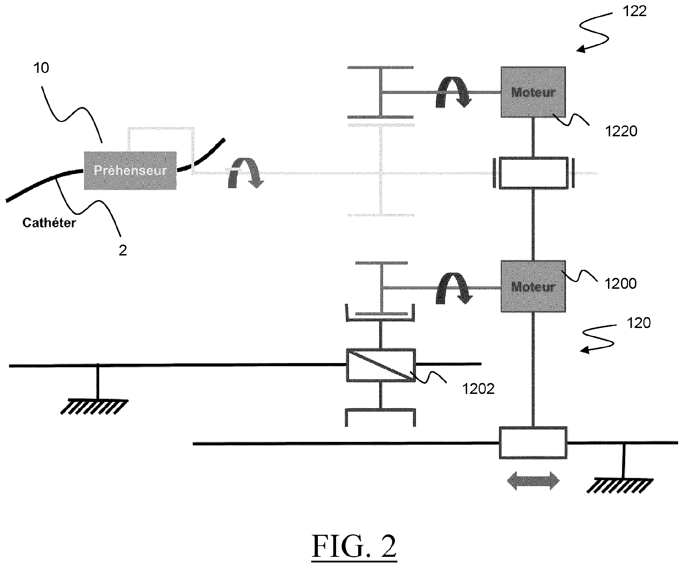

FIG. 2 illustrates an example of a diagram of the kinematic links of the robotic catheter manipulator. The pneumatic gripper 10 is represented with a catheter inserted in the cavity. The first actuator 120 comprises a first motor 1200 that is used to generate the force for performing the translation and the second actuator 122 comprises a second motor 1220 to generate the torque for performing the rotation. In this example, the transmission and transformation of the rotation of the first motor 1200 into translation is achieved by a nut-and-spindle system 1202. Still in this example, two toothed wheels are used to transmit the rotational movement of the second motor 1220 to the pneumatic gripper 10; the driving toothed wheel is fixed to the motor output shaft, and the driven toothed wheel is fixed to the pneumatic gripper 1.

In examples, the robotic catheter manipulator may comprise a body comprising at least one guide (also referred to as rail) forming the first axis; the pneumatic gripper is movable along the said at least one guide. In this situation, the direction of the longitudinal axis of the elongated medical device is substantially the same as the direction of at least one guide forming the first axis. In the example shown on FIG. 2, the translation of the pneumatic gripper 10 is guided by a rail.

The number of guide of the body of the robotic catheter manipulator may vary. In an example, the body of the robotic catheter manipulator may comprise one guide passing through the pneumatic gripper on both sides, so that the pneumatic gripper cannot be disengaged from the rail. The rail may have any type of profile, e.g. a tube, a T profile... In another example, the body of the robotic catheter manipulator may comprise two guides that may be parallel one each other. Any type of profile may be used. Two rails instead of one provides more securely maintaining the pneumatic gripper about the body of the robotic catheter manipulator. In further examples, three or more guides may be used.

In examples, the pneumatic gripper movable along the said at least one guide may refer to the pneumatic gripper in contact with the one or more guide, or it may refer to the positioning system, on which the pneumatic grippe is maintained, in contact with the one or more guide. In other words, the pneumatic gripper or the positioning system may be directly guided by the guide(s).

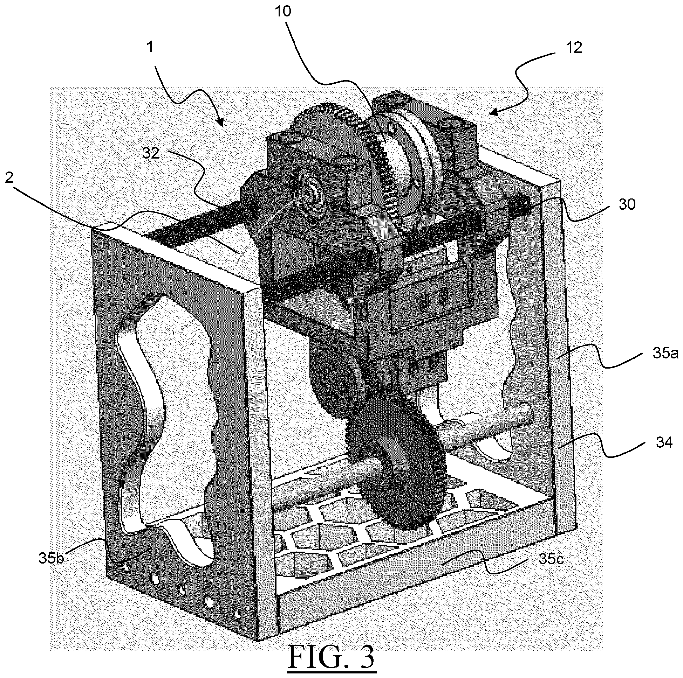

FIG. 3 shows an example of the robotic catheter manipulator 1. In this example, the robotic catheter manipulator 1 has a body 34 that comprises a base 35c and two walls 35a and 35b of the body. The body further comprises two guides 30, 32 that are maintained by and located between the two walls 35a and 35b. The two guides (that are also referred to as rails) pass through the positioning system 12 on which the pneumatic gripper is maintained. In this example the guides are tubes and the positioning system 12 has at least one through hole for each guide. In FIG. 3, the positioning system has two though holes per guide; this improves the displacement (one could say the slide) of the positioning system 12. The positioning system travels between two extreme positions that are defined respectively by the walls 35a and 35b.

Still in FIG. 3, the positioning system comprises a cradle (one could say the positioning system is cradle-shaped), on which the pneumatic gripper system is held: the pneumatic gripper is held at both ends on the cradle formed by the positioning system, for example via a rotational connection at each end. Thus, the pneumatic gripper maintained on the positioning system can freely rotate, being understood that the rotation is controlled by the positioning system, e.g. the second actuator 122.

In examples, at least one of the first and second actuators may be a compressed-air stepper motor, which is a type of pneumatic motor that combines the principles of a stepper motor with the use of compressed air as its driving force. A compressed-air stepper motor uses air pressure to control the motor's steps and movement. A compressed-air stepper motor can be used in high electromagnetic interference environments such as a magnetic resonance imaging (MRI) environment.

In examples, at least one of the first and second actuators may be an electric motor. In an example, the electric motor is a stepper motor that allows precise control of position of the motor.

In examples, the robotic catheter manipulator may comprise a clamp for securing one end of the elongated medical device, e.g. a catheter. As already discussed, the proximal portion of the elongated medical device is the end of the elongated medical device that is kept outside the blood vessels. When the elongated medical device distal portion has been placed into the vessels of the patient, the proximal portion may be clamped to the robotic catheter manipulator so that the elongated medical device cannot be moved anymore; being understood that the robotic catheter manipulator is not moved. For example, once the intermediate catheter has been placed into the patient's body, the proximal portion of the elongated medical device is clamped while the microcatheter is inserted into the patient's blood vessels.

In examples, the materials of the robotic catheter manipulator are made of MRI (Magnetic resonance imaging)-safe materials. MRI-Safe materials are materials that pose no risk in the MRI environment. They are non-magnetic and do not interact with the magnetic field in any way. In examples, MRI-Safe materials may be, but are not limited to, (1) non-magnetic metals such as titanium, aluminum, copper, brass, (2) polymers such as polycarbonate, polyethylene, polytetrafluoroethylene (also known as Teflon^{™}) that may be used in coatings for MRI-safe medical instruments and guidewires, polyvinyl chloride, polypropylene, acrylic (PMMA): Used for MRI-safe barriers and some equipment covers due to its clarity and durability, (3) ceramics and glass such as borosilicate glass, alumina, (4) non-conductive materials such as silicone, Teflon^{™}...

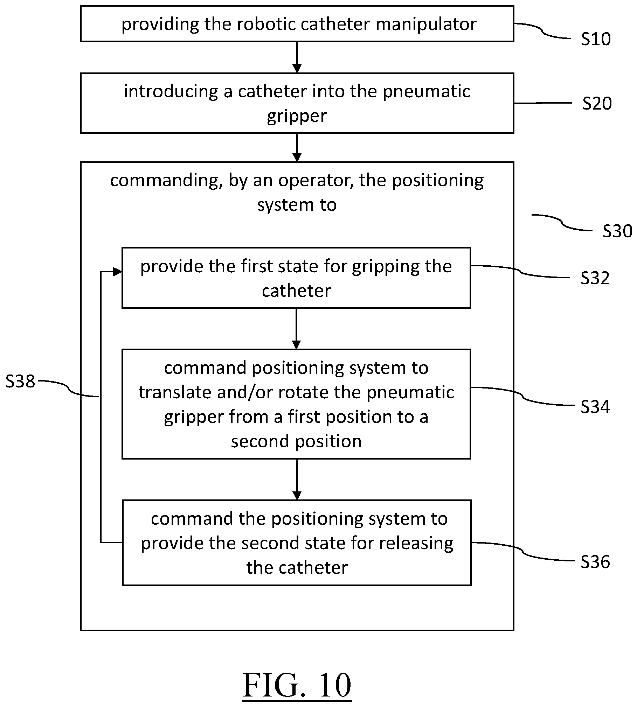

Referring now to FIG. 10, is discussed a method of robotically guiding an elongated medical device such as a catheter.

At S10, a robotic catheter manipulator according to the invention is provided. The provided robotic catheter manipulator may be one according to any combination of the examples that have been discussed. Providing may means selecting the robotic catheter manipulator.

At S20, an elongated medical device (e.g. a catheter) is introduced into the pneumatic gripper. Introducing into the pneumatic gripper refers to arranging the elongated medical device with the pneumatic gripper in such a way that the pneumatic gripper can grip the elongated medical device if the pneumatic gripper provides the first state. An example of introducing the elongated medical device in the pneumatic gripper is represented on FIG. 3 where the elongated medical device 2 has been placed in the cavity of the pneumatic gripper 10. The introduction of the elongated medical device in the pneumatic gripper may be done by an operator (e.g. a physician) of the robotic catheter manipulator.

Then, at S30, the operator commands the positioning system to perform the steps S32 to S36, and possibly S38. Commanding the positioning system means that the operator controls the manipulator operations, e.g. using a control interface that allow the operator to interact with the manipulator, guiding its actions and behaviors.

In examples, the control interface may comprise a physical manual interface where the operator manually inputs commands to control the manipulator directly. A physical manual interface may be, but is not limited to, a joystick controller, a handheld device that provide a direct interface to control the manipulator.

In examples, the control interface may comprise a graphical user interface (GUI) providing graphical elements, like buttons, icons, and menus, to interact with the manipulator. The GUI is typically displayed on a computer/tablet/smartphone screen.

In examples, the control interface may comprise a text-based interface that uses text input to control the robot, e.g. command-line interfaces (CLI), scripting languages written in programming languages such as Python, C++, or JavaScript. Scripting allows for complex behaviors and automation of repetitive tasks, Robot Operating System (ROS) commands; ROS is a popular framework for robotics development.

In examples, the control interface may comprise a Natural User Interface (NUI) that allows users to control the manipulator in ways that are more intuitive and natural, often using sensors to detect human movements or speech. For example, voice commands allow commanding the manipulator with the operator's voice. As another example, the manipulator may be controlled using hand gestures or body gestures recognized by cameras or motion sensors. Gesture control is useful in environments where hands-free operation is needed, especially in the context of neurointervention.

In examples, the control interface may comprise a haptic interface that provides tactile feedback to the user, enhancing control precision through the sense of touch. For example, a haptic interface may be a force-feedback joysticks and gloves that provide feedback to the user based on the robot's interaction with the environment. In the context of interventional neuroradiology, the physician can feel the movements of the elongated medical device, allowing for its delicate manipulation.

In examples, the control interface may comprise an augmented reality (AR) interface that use devices like AR glasses or tablets to overlay digital information on the physical world.

In examples, the control interface may comprise a machine learning interface where a model has been trained to adjust the manipulator's behavior. Such an interface increases the autonomy of the manipulator.

In examples, the control interface may comprise an endovascular simulator, which is a physical tool that registers in real-time the translations and rotations of elongated medical devices.

At S32, the operator commands the robotic catheter manipulator to provide the first state for gripping the elongated medical device.

At S34, the operator commands the positioning system on which the pneumatic gripper is maintained to move the pneumatic gripper from a first position to a second position. The movement may be a translation or a rotation or a combination of translation and rotation. The translation and rotation have been discussed in reference to the first and second actuators; this discussion applies.

At S36, the operator commands the robotic catheter manipulator to provide the second state for releasing the elongated medical device. The elongated medical device is no more gripped by the pneumatic gripper.

Then, at S38, the operations of S32, S34 and S36 are repeated. Several repetition may be performed until the elongated medical device is positioned as requested by the operator.

It is to be understood that if the movement of the positioning system at 36 comprises a translation, then the repetition S38 may also comprise, before S32, (i) a translation of the positioning system in the direction of the first position (thus from the second to the first position); this is performed is the operator wishes to translate again the elongated medical device from the first to the second positions. Inversely, (ii) no translation of the positioning system to the direction of the first position is performed if the operator wishes to move in the opposite direction the elongated medical device, e.g. from the second to the first positions.

It is to be understood that if the movement of the positioning system at 36 comprises a rotation, then the repetition S38 may also comprise, before S32, (iii) a rotation of the positioning system to the initial position, for example a counterclockwise rotation after a clockwise rotation. The inlet and outlet fluidly connected with the pneumatic gripper may limit the rotation angle of the positioning system and recovery of the initial position may be necessary.

In examples, the elongated medical device may be a telescopic elongated medical device that comprises two or more tubes with a range of diameters that fit within each other. The method of robotically guiding a catheter further comprises providing a robotic catheter manipulator for each of the two or more tubes. Each tube forming the telescopic elongated medical device is thus associated with one robotic catheter manipulator. The operations S32, S34, S36 and S38 are performed form each tube of the telescopic elongated medical device, but starting with the with the largest diameter tube and ending with the smallest diameter tube. For example, if a telescopically mounted catheter comprises a guide catheter, an intermediate catheter and a microcatheter, the guide catheter will be positioned first by its robotic catheter manipulator, in a second step the intermediate catheter by its own robotic catheter manipulator, and finally the microcatheter by its robotic catheter manipulator. As already discussed, a robotic catheter manipulator may comprise a clamp for securing one end of the catheter; preferably, a tube of the telescopic catheter is clamped before the next tube (with a smaller diameter) which is guided by a robotic catheter manipulator. The operation S32, S34, S36 and S38 can be performed for each elongated medical devices at any moment of the procedure. This is possible thanks to the reconfigurable design of the catheter manipulator. For example, this makes it possible to adjust the position of an intermediate catheter when required during the procedure in order to facilitate navigation of microcatheter and/or micro-guidewire.

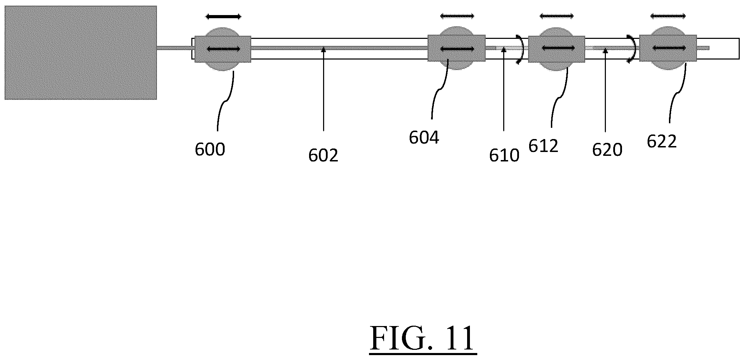

Referring now to FIGs. 11 to 14, an example of the method is illustrated where each of the robotic catheter manipulator is comprised on (that is secured on) a robotic arm providing up to six degrees of freedom. The set of robotics arms form a robotic catheter placement system. The system further comprises a control unit that configures the position of the one or more robotic arms, the selection of the first and second states for each robotic catheter manipulator and the positioning system of each robotic catheter manipulator. In these examples, the elongated medical device is telescopic and comprises one guide catheter 602, one intermediate catheter 610, and one microcatheter 620.

The robotic arms are arranged to mimic an operator (for instance a physician) stance, making it possible for the delivery of elongated medical device, e.g. a telescopic catheter. The robotic arms aim at mimicking the arms of the operator, while the robotic catheter manipulators aim at mimicking the hand and fingers of the operator. More precisely, the pneumatic gripper is a simulation of the fingers of the operator and the positioning system is a simulation of the hands of the operator. The robotic arms prevent the bouncing of the elongated medical device during its manipulations by the robotic catheter manipulators, thus preventing patient injuries when guiding the elongated medical device in the body of the patient.

As already explained, the pneumatic actuator can mimic the thumb-index pinch. Together with the positioning system, the elongated medical device can be pushed and retreated in the blood vessels. But as the catheter (e.g. a tube of a telescopic catheter) enters the vessels, the proximal end of the catheter gets closer and closer to the robotic catheter manipulator if the position of the robotic catheter manipulator about the body of the patient is unchanged; it is no more possible to enter the elongated medical device in the vessel. In order to avoid this situation, the arm can modify the position of the robotic catheter manipulator (for example toward the body of the patient) so that the proximal end of the catheter is once again moved away from the robot, the catheter can continue to penetrate the patient's body upon actions of the robotic catheter manipulator. In examples, the pneumatic gripper of the robotic catheter manipulator may be in the second state while the arm of the robotic catheter placement system changes position; indeed, the purpose of the arm is not to make the catheter entering in the vessels but to place the robotic catheter manipulator in a position allowing the manipulation of the catheter.

FIG. 11 shows an example of initial state of a robotic catheter placement system that comprises four arms, each arm providing up to six degrees of freedom. Each arm comprises a robotic catheter manipulator of the present disclosure, 600, 604, 612, 622. The telescopic elongated medical device has been inserted in the robotic catheter manipulators. As shown by the arrows, each robotic catheter manipulator can be displaced about an axis of the telescopic elongated medical device, or respectively displaced about a respective axis of one of the guides 602, 610, 620. Each arm of the robotic catheter placement system can be moved about the axis of the telescopic elongated medical device, or respectively displaced about the respective axis of one of the guides 602, 610, 620.

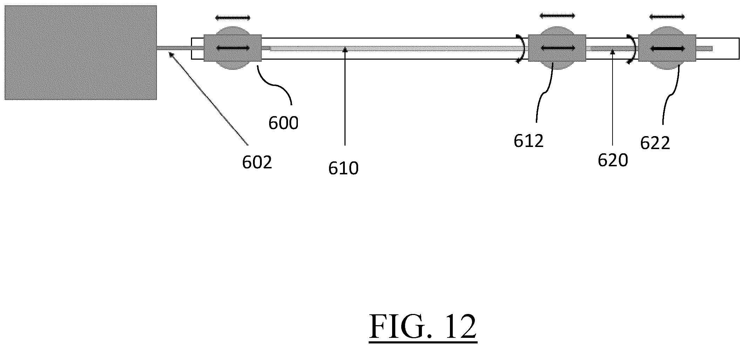

FIG. 12 shows an example of a second state that follows the initial state of FIG. 11. The catheter 602 has been inserted in the blood vessel of the body of the patient and has reached its final position: this position will be maintained for the next states. Thus, the robotic arm that comprises the robotic catheter manipulator 600 now holds a position in the space that will not change also; the same of the robotic catheter manipulator 600. The proximal end of the catheter 602 may be clamped on the robotic catheter manipulator 600 securing the distal end of the catheter 602.

Still in FIG. 12, the robotic catheter manipulator 612 performs translation and rotation movements for inserting the catheter 610. The robotic arm holding the robotic catheter manipulator 612 moves as the catheter 610 is inserted so that the robotic catheter manipulator 612 can manipulate the catheter 610. The arm that holds the robotic catheter manipulator 622 may be also displaced so that the robotic catheter manipulator 622 follows the displacement of the robotic catheter manipulator 612; the robotic catheter manipulator 622 may also performed translation and rotation movements while being displaced by its arm.

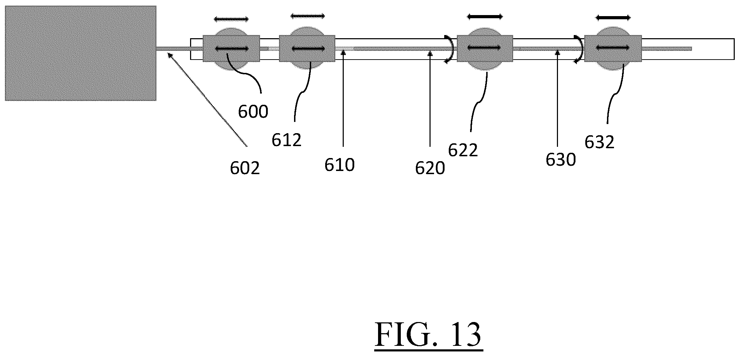

Referring now to FIG. 13, is represented a third state where the catheter 610 has been inserted. The catheter 610 may be clamped on the robotic catheter manipulator 612 as it has reached it final position. The catheter 620 is now inserted upon translation and rotation movements of the robotic catheter manipulator 622. The arm holding the robotic catheter manipulator 622 is also displaced for ensuring that at least the proximal end of the catheter 620 can be manipulated by the robotic catheter manipulator 622. FIG. 13 further shows an interventional tool 630 (e.g. a stent retriever) that will be inserted also. As shown on FIG. 13, the robotic catheter manipulator 632 intended to manipulate the interventional tool may also be displaced so that the robotic catheter manipulator 632 follows the displacement of the robotic catheter manipulator 622; the robotic catheter manipulator 632 may also performed translation and rotation movements while being displaced by its arm.

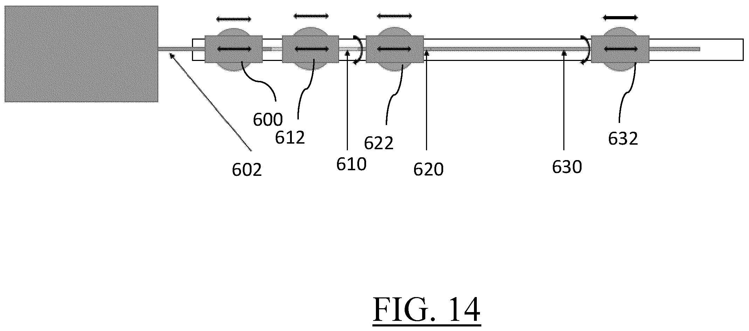

FIG. 14 shows a fourth state where the catheter 620 has been inserted and has reached its final position. The distal end of the catheter 620 may be clamped on its robotic catheter manipulator 622. The interventional tool 630 is now manipulated by the robotic catheter manipulator 632 so that the interventional tool will reach the intervention zone. The arm holding the robotic catheter manipulator 632 accompanies the displacement of the catheter 630.

As shown in the example of FIGS. 11 to 14, the robotic catheter manipulators imitate the advancement, retraction, rotation and clamping of human hands (especially neurointerventionists) and combinations of them, and the arm imitate the movements of the human arm. FIGS. 11 to 14 are similar, except that each of these figures shows the displacement of a given arm. These figures thus illustrate the method illustrated on FIG. 10, except that the positioning system further displaces the arms of the robotic catheter manipulator.

In example, the control unit may comprise the control interface discussed in reference to S30.

In examples, the robotic catheter placement system may provide one or more remote communication modules for remotely controlling the control interfaces of the system. It is to be understood that any technology adapted for remote transmission of commands may be used, e.g. wireless control interfaces, Bluetooth^{©} or Wi-Fi Controllers, Radio Frequency (RF) Controllers, Cloud-Based Control.

In example, the control unit may comprise a computer program processing unit and a memory storing instructions that cause the command unit to configure the position of the one or more robotic arms, configure the selection of the first and second states for each of the said one robotic catheter manipulator, and configure the positioning system. The computer program may ensure that the command unit executes the commands in real-time. Real time means that data is processed as soon as it is inputted, often with minimal delay, so that the system responds quickly and reliably.

The system is partly is computer-implemented. This means that steps of the method performed by the system are executed by at least one computer, or any system alike. Thus, the system can operate fully automatically, or, semiautomatically.

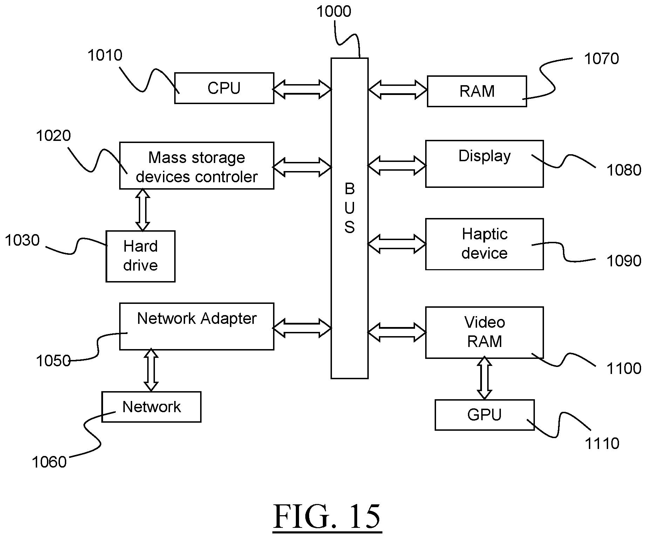

FIG. 15 shows an example of the command unit of the system, wherein the command unit of the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A robotic catheter manipulator, comprising:
- a pneumatic gripper, the pneumatic gripper providing a first state for gripping a catheter and a second state for releasing the catheter; and
- a positioning system on which the pneumatic gripper is maintained.

2. The robotic catheter manipulator of claim 1, wherein the pneumatic gripper comprises a cavity adapted to receive the catheter on a portion thereof, the cavity forming a wall around the portion of the catheter, the wall comprising one or more pneumatic cushions; and
wherein the pneumatic gripper provides the first state when the one or more pneumatic cushions are inflated and provides the second state when the one or more pneumatic cushions are deflated.

3. The robotic catheter manipulator of claim 3, wherein the one or more pneumatic cushions are fluidly interconnected.

4. The robotic catheter manipulator of claim 2 or 3, wherein the pneumatic gripper has an elongated shape, preferably the elongated shape is a cylinder; and
wherein the cavity adapted to receive the catheter on a portion thereof has an elongated shape with a diameter comprises between 1mm and 3mm, preferably between 1.5mm and 1.85mm.

5. The robotic catheter manipulator of any one of claims 2 to 4, wherein the cavity is substantially a cylindric, the cavity form a through-hole, and the wall comprising one or more pneumatic cushions has a thickness which is substantially the same along its length.

6. The robotic catheter manipulator of anyone of claims 1 to 5, further comprising an inlet and an outlet fluidly connected with the pneumatic gripper, preferably the inlet and outlet being merged.

7. The robotic catheter manipulator of anyone of claims 1 to 6, wherein the positioning system comprises a first actuator for displacing the pneumatic gripper about a first axis and a second actuator for rotating the pneumatic gripper about the first axis.

8. The robotic catheter manipulator of claim 7, wherein the robotic catheter manipulator further comprises a body comprising at least one guide forming the first axis, the pneumatic gripper being movable along the guide.

9. The robotic catheter manipulator of claim 7 or 8, wherein the first and second actuators comprise compressed-air stepper motors or others electric motors.

10. The robotic catheter manipulator of anyone of claims 1 to 9, wherein the robotic catheter manipulator further comprises a clamp for securing one end of the catheter.

11. A method of robotically guiding a catheter, comprising:
- providing the robotic catheter manipulator of any one of claims 1 to 10;
- introducing a catheter in the pneumatic gripper;
- commanding, by an operator, the positioning system to:
-- provide the first state for gripping the catheter;
-- command positioning system to translate and/or rotate the pneumatic gripper from a first position to a second position;
-- command the positioning system to provide the second state for releasing the catheter; and
- repeating the commanding of the positioning system until the catheter is positioned as requested by the operator.

12. The method according to claim 11, wherein the catheter is a telescopic catheter comprising two or more tubes with a range of diameters that fit within each other, further comprising:
- providing a robotic catheter manipulator for each of the two or more tubes;
- commanding successively, by an operator, the positioning system for each tube of the telescopic catheter, starting with the largest diameter tube and ending with the smallest diameter tube.

13. A robotic catheter placement system, comprising:
- one or more robotic arms providing up to six degrees of freedom, each of the one or more robotic arms comprising one robotic catheter manipulator according to any one of claims 1 to 10;
- a command unit for configuring:
- - the position of the one or more robotic arms;
- - the selection of the first and second states for each of the said one robotic catheter manipulator; and
- - the positioning system.

14. The robotic catheter placement system of claim 13, wherein the control unit comprises a processing unit and a memory storing instructions that, when executed by the processing unit, cause the command unit to configure the position of the one or more robotic arms, configure the selection of the first and second states for each of the said one robotic catheter manipulator, and configure the positioning system.

15. A computer program comprising instructions for causing in real-time the command unit of claim 14 to configure the position of the one or more robotic arms, configure the selection of the first and second states for each of the said one robotic catheter manipulator, and configure the positioning system.
